# EUROPEAN PATENT APPLICATION

(11) **EP 4 205 806 A1**
(43) Date of publication of application: **05.07.2023**
(21) Application number: 22150038.2
(22) Date of filing: 03.01.2022
(51) Int. Cl.: A61N 1/375, A61N 1/05

(54) **PORT FOR A LEADLESS PACEMAKER**

(71) Applicant: BIOTRONIK SE & Co. KG, 12359 Berlin (DE)
(72) Inventor: Podszuck, Christiane, 61273 Wehrheim (DE); Muentjes, Jutta, 91056 Erlangen (DE)
(74) Representative: Biotronik Corporate Services SE

(57) **Abstract**

A pacemaker system comprising a port (40) for a leadless pacemaker (50) and a leadless pacemaker (50) for stimulating cardiac activity of a heart of a patient is provided. The port (40) comprises: a first connector (44), which is arranged at a first side of the port (40) and which is configured for mechanically and detachable attaching the leadless pacemaker (50) to the port (40); and at least one anchoring member (30), which is mechanically coupled to the first connector (44), for anchoring the port (40) to tissue of the heart. The leadless pacemaker (50) comprises: a second connector (54), which is arranged at a first side of the leadless pacemaker (50) and which is configured for mechanically and detachable attaching the leadless pacemaker (50) to the port (40); a stimulation circuit (32), which is configured for generating a stimulation signal; and an energy source (34), which is electrically coupled to the stimulation circuit (32) and which is configured to supply energy to the stimulation circuit (32).

## Description

The present invention relates to a pacemaker system. Particularly, the present invention relates to a port for a leadless pacemaker and to the leadless pacemaker for stimulating cardiac activity of a heart of a patient. Furthermore, the present invention relates to a detaching tool for detaching the leadless pacemaker from the port, and to a method for exchanging the leadless pacemaker.

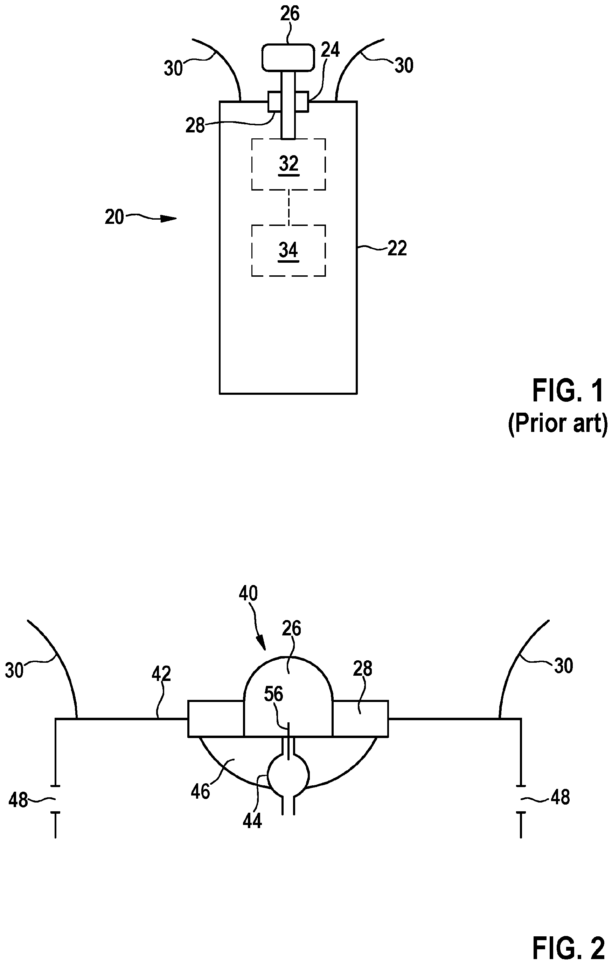

As shown in figure 1, a conventional leadless pacemaker 20 consists of a conventional housing 22, a stimulation circuit 32 for generating a stimulation signal, a stimulation electrode 26, which is isolated against the conventional housing 22 by an insulation 28, a feedthrough 24 extending through the conventional housing 22 for transferring the stimulation signal from the stimulation circuit 32 to the stimulation electrode 26, an energy source 34, e.g. a battery, and at least one anchoring member 30, e.g. a tine, to fix the conventional leadless pacemaker 20 in the heart tissue.

If the conventional leadless pacemaker 20 is implanted in the heart of a patient and does not work properly anymore, e.g. because the stimulation circuit 32 has a malfunction and/or because the energy source 34 is exhausted, the conventional leadless pacemaker 20 may be removed and substituted by another leadless pacemaker. Although a catheter examination may be acceptable all 5 years or even less, the battery is conventionally designed for a ten-year life expectation to avoid the elaborate retrieval process. Therefore a capacity of the battery needs to be large. In turn, the battery needs to be rather large.

It has been observed that the retrieval of the conventional leadless pacemaker 20 from heart tissue includes the risk of tissue violation. Although successful explantations have been reported, a behaviour of an encapsulation (not shown) of the conventional leadless pacemaker is unpredictable and may be a challenge for the retrieval process. For example, if the encapsulation is damaged during explantation, the encapsulation or parts of the encapsulation may stay within the body of the patient and may cause harm to the patient. If the explantation is possible, the same spot in the tissue cannot be used again for stimulation due to tissue violation and fibrosis.

If the explantation is considered as too risky, another leadless pacemaker may be implanted next to the conventional leadless pacemaker 20, e.g. after depletion of the energy source 34. In particular, the conventional leadless pacemaker 20 may be left in the heart tissue without being used anymore and the other leadless pacemaker 20 may be implanted in addition to the conventional leadless pacemaker 20. In other words, a replacement pacemaker may be implanted on a new implantation site. This might be challenging due to the limited space, mechanical interaction of the old and the new leadless pacemaker 20 and the fact that a good position, in many cases the best position, for stimulation is already occupied by the conventional leadless pacemaker 20.

Due to the above-mentioned reasons, the patient may not fully profit from the expected development of leadless pacers in the future and the application of leadless pacemakers may be limited.

It is an object of the present invention to provide an improved pacemaker system, which provides an improved possibility of replacing implanted leadless pacemakers and which particularly enables a high probability of successfully explanting and replacing the leadless pacemaker with only a few or without any tissue violation of the heart of the patient. Further, it is an object of the present invention to provide an improved detaching tool and an improved method for exchanging a leadless pacemaker, which provide an improved possibility of replacing implanted leadless pacemakers and which particularly enable a high probability of successfully explanting and replacing the leadless pacemaker with only a few or without any tissue violation of the heart of the patient.

Such objects may be met with the subject-matter of the independent claims. Advantageous embodiments are defined in the dependent claims as well as the corresponding specification and figures.

According to a first aspect of the present invention, a port for a leadless pacemaker for stimulating cardiac activity of a heart of a patient is proposed. The port comprises a first connector, which is arranged at a first side of the port and which is configured for mechanically and detachable attaching the leadless pacemaker to the port; and at least one anchoring member, which is mechanically coupled to the first connector, for anchoring the port to tissue of the heart.

According to a second aspect of the present invention, the leadless pacemaker for stimulating cardiac activity of the heart of the patient is provided. The leadless pacemaker comprises: a second connector, which is arranged at a first side of the leadless pacemaker and which is configured for mechanically and detachable attaching the leadless pacemaker to the port for the leadless pacemaker, the port being attached to tissue of the heart; a stimulation circuit, which is configured for generating a stimulation signal, and an energy source, which is electrically coupled to the stimulation circuit and which is configured to supply energy to the stimulation circuit.

According to a third aspect of the present invention, a pacemaker system comprising the above port and the above leadless pacemaker is provided.

According to a fourth aspect of the present invention, a detaching tool for detaching the leadless pacemaker from the port, which is attached to the tissue of the heart of the patient, is provided. The detaching tool comprises: releasing means, which are configured for interacting with fixation means at a housing of the leadless pacemaker such that the fixation means may be released by the releasing means; engaging means, which are coupled to the releasing means and which are configured to engage with retrieval means of the leadless pacemaker such that the leadless pacemaker may be removed from the port by the detaching tool, and coupling means, which are coupled to the releasing means and/or the engaging means, and which are configured for coupling the detaching tool to a catheter.

According to a fifth aspect of the present invention, a method for exchanging the leadless pacemaker, which is attached to the port, the port being attached to tissue of the heart of the patient, is provided. The method comprises: engaging the leadless pacemaker and releasing fixation means, with which the leadless pacemaker is mechanically coupled to the port; removing the leadless pacemaker from the port; and coupling another leadless pacemaker to the port. Before engaging the implanted leadless pacemaker, the tissue of the patient may be opened until the leadless pacemaker is exposed.

Ideas underlying embodiments of the present invention may be interpreted as being based, inter alia, on the following observations and recognitions.

Briefly summarised in a non-limiting manner, embodiments of the present invention relate to a splitting of a conventional leadless pacemaker into two parts: the port, which may be permanent and/or implantable, which comprises the anchoring member and which comprises the first connector, the first connector and the second connector forming a plug-in mechanism for the actual leadless pacemaker, and the actual leadless pacemaker, which comprises the housing of the leadless pacemaker, the energy source and the stimulation circuit. The leadless pacemaker may be linked via its second connector to the first connector of the port. The leadless pacemaker and the port may be implanted together, wherein the connectors form a non-permanent, reversible fixation mechanism, such that the leadless pacemaker may be explanted without the port later. A stimulation electrode for stimulating the cardiac activity may be arranged at the port or at the leadless pacemaker. After depletion of the energy source or in case of a failure of the leadless pacemaker, the leadless pacemaker may be decoupled from the port in a catheter examination with a special removal tool, e.g. the above detaching tool. After removal of the old leadless pacemaker, the new leadless pacemaker may be attached to the existing port with a special re-implantation tool. A signal transfer from the leadless pacemaker to the port may be achieved e.g. via an insulated plugin mechanism or via ultrasonic sound and piezo transducers.

The above solution facilitates the exchange of old and new leadless pacemakers inside the body. Several advantages result from the above construction: The port does not have to be explanted, wherein fibrosis and an encapsulation around the port do not affect the exchange of the leadless pacemaker. The new leadless pacemaker does not need to be implanted next to the old leadless pacemaker. Additionally, the leadless pacemaker does not need any feature, which allows to deactivate the implanted leadless pacemaker from outside the body as it can be easily removed. An optimum stimulation point in the heart tissue, at which the port is positioned, may be reused with the new leadless pacemaker. The capacity of the energy source may shrink as an exchange of the device within a catheter examination earlier than 10 years might become acceptable. The patient may profit from the expected development of leadless pacemakers in the future. The above split-design might allow a greater range of application of the leadless pacemaker, e.g. in younger patients etc.

The anchoring member may comprise one or more tines, wires, sheets or tubes. The energy source may be a battery. The first connector may interact with second connector of leadless pacemaker by a press fit, interference fit, positive fit, and/or bayonet mount. That the leadless pacemaker is attached to the port means that the second connector is connected to the first connector. That the leadless pacemaker is detachable attached to the port means in this application that it is attached in a reversible releasable manner, in particular in a nondestructive manner. The leadless pacemaker may be attached to or may be detached from the port by a corresponding engaging tool and/or, respectively, by the above detaching tool.

In the following, characteristics of embodiments of the present invention will be described in more detail.

According to a preferred embodiment, the port comprises: the stimulation electrode at a second side of the port, the second side facing away from the first side; and a receiver unit, which is electrically coupled to the stimulation electrode and which is configured for receiving a stimulation signal from the leadless pacemaker and to forward the stimulation signal to the stimulation electrode, wherein the stimulation electrode is configured to stimulate the cardiac activity depending on the stimulation signal. So, the stimulation electrode may be a part of the port and may stay within the body of the patient, when the leadless pacemaker is exchanged. This may contribute to that the stimulation electrode may be positioned at a very good, e.g. the optimal position for the stimulation. If the port is implanted in the tissue of the heart, the second side of the port faces the tissue of the heart. The stimulation electrode may be insulated against the anchoring member. The receiver unit may be a wire, an electric socket or plug, with the leadless pacemaker comprising the corresponding wire terminal, electric plug or, respectively, socket, or, in case of a wireless stimulation signal, a wireless receiver. The stimulation signal may be an ultrasonic or an electromagnetic, i.e. inductive, signal.

According to a preferred embodiment, the stimulation signal from the leadless pacemaker is the wireless signal and the receiver unit is the wireless receiver configured for receiving the wireless stimulation signal. The usage of the wireless signal for transmitting the stimulation signal enables to completely close the housing of the leadless pacemaker against the environment. In particular, no feedthroughs through the housing of the leadless pacemaker are necessary to feed any wires through the housing of the leadless pacemaker. This contributes to that no dirt or body fluids may enter the leadless pacemaker. The wireless signal may be an electromagnetic signal or an ultrasonic signal. The wireless receiver correspondingly is a radio receiver or an ultrasonic receiver. Generally, the ultrasonic receiver may be any component which, due to their physical characteristics, enables emitting and receiving ultrasonic acoustic signals. For example, the ultrasonic transmitter and receiver may be micro-electromechanical systems (MEMS). For example, the ultrasonic transmitter and/or the ultrasonic receiver comprise or are piezoelectric micromachined ultrasonic transducers (PMUT).

According to a preferred embodiment, the port comprises a housing, which surrounds a cavity for accommodating at least a part of the leadless pacemaker and which has a recess extending through the housing and in which at least a part of the first connector and/or the stimulation electrode are arranged, and/or an insulation, which is coupled to the housing and which is configured to electrically insulating the stimulation electrode against the housing. The housing of the port may contribute to an accurate positioning of the leadless pacemaker at the tissue and a secure attachment of the leadless pacemaker to the port.

According to a preferred embodiment, the anchoring member is arranged at the housing. The anchoring member at the housing of the port may contribute to the secure attachment of the port to the tissue.

According to a preferred embodiment, the leadless pacemaker comprises a transmitter unit, which is electrically coupled to the stimulation circuit and which is configured for sending the generated stimulation signal to the receiver unit of the port. The transmitter unit may be a wire, or an electric socket or plug, with the port comprising the corresponding wire terminal, electric plug or, respectively, socket, or, in case of a wireless stimulation signal, a wireless sender.

According to a preferred embodiment, the stimulation signal is the wireless signal and the transmitter unit is the wireless sender configured for wirelessly sending the stimulation signal to the receiver unit of the port. The stimulation signal may be the ultrasonic or electromagnetic, i.e. inductive, signal. The usage of the wireless signal for transmitting the stimulation signal enables to completely close the housing of the leadless pacemaker against the environment. In particular, no feedthroughs through the housing of the leadless pacemaker are necessary to feed any wires through the housing of the leadless pacemaker. This contributes to that no dirt or body fluids may enter the leadless pacemaker. The wireless signal may be an electromagnetic signal or an ultrasonic signal. The wireless sender correspondingly is a radio sender or an ultrasonic sender. Optionally, e.g. in case of the wireless sender, the energy source may be electrically coupled to the transmitter and may be configured to supply energy to the transmitter.

According to a preferred embodiment, the leadless pacemaker comprises the stimulation electrode at the first side of the leadless pacemaker, wherein the stimulation electrode is electrically coupled to the stimulation circuit for receiving the stimulation signal and wherein the stimulation electrode is arranged and configured such that the cardiac activity of the heart of the patient can be stimulated by the stimulation electrode, if the port is attached to the tissue of the heart and if the leadless pacemaker is mechanically coupled to the port. Providing the stimulation electrode at the leadless pacemaker enables to completely close the housing of the leadless pacemaker against the environment. In particular, no feedthroughs through the housing of the leadless pacemaker are necessary to feed any wires through the housing of the leadless pacemaker for transmitting the stimulation signal. This contributes to that that no dirt or body fluids may enter the leadless pacemaker.

According to a preferred embodiment, the leadless pacemaker comprises a housing and fixation means, which are arranged at the housing and which are configured for detachable attaching the housing to the port. The fixation means at the housing of the leadless pacemaker provide a securement of the leadless pacemaker to the port and as such to the heart in addition to the fixation via the connectors and the anchoring member of the port. The fixation means may comprise one or more of the group of tines, wires, sheets, and tubes. The tines, wires, sheets, or tubes provide a secure, simple and cost-efficient attachment of the leadless pacemaker to the port.

According to a preferred embodiment, the leadless pacemaker comprises: retrieval means at a second side of the leadless pacemaker, the second side of the leadless pacemaker facing away from the first side of the leadless pacemaker. The retrieval means enable a quick and simple retrieval of the leadless pacemaker from the port. The retrieval means may comprise one or more of the group of a sling, knob, loop, eyelet. The sling, knob, loop, or eyelet contribute to provide the retrieval means in a simple and cost-efficient way.

According to a preferred embodiment, the leadless pacemaker is mechanically and detachable attached to the port. In other words, the second connector of the leadless pacemaker is mechanically and detachable coupled to the first connector of the port.

It shall be noted that possible features and advantages of embodiments of the invention are described herein with respect to various embodiments of the port, the leadless pacemaker, and the pacemaker system, on the one hand, and with respect to various embodiments of the detaching tool, on the other hand, as well as with respect to corresponding method. One skilled in the art will recognize that the features may be suitably transferred from one embodiment to another and features may be modified, adapted, combined and/or replaced, etc. in order to come to further embodiments of the invention.

In the following, advantageous embodiments of the invention will be described with reference to the enclosed drawings. However, neither the drawings nor the description shall be interpreted as limiting the invention.
- Fig. 1: shows an example of a conventional leadless pacemaker.
- Fig. 2: shows a port for a leadless pacemaker according to an embodiment of the present invention.
- Fig. 3: shows a partial view of a pacemaker system according to an embodiment of the present invention.
- Fig. 4: shows a pacemaker system according to an embodiment of the present invention.
- Fig. 5: shows a pacemaker system according to an embodiment of the present invention.
- Fig. 6: shows a partial view of a pacemaker system according to an embodiment of the present invention.
- Fig. 7: shows a pacemaker system and a delivery tool according to an embodiment of the present invention.
- Fig. 8: shows a pacemaker system and a detaching tool according to an embodiment of the present invention.
- Fig. 9: shows a pacemaker system and a re-implanting tool according to an embodiment of the present invention.
- Fig. 10: shows a pacemaker system according to an embodiment of the present invention.
- Fig. 11: shows a flow diagram of a method for exchanging a leadless pacemaker according to an embodiment of the present invention.

The figures are only schematic and not to scale. Same reference signs refer to same or similar features.

Fig. 1 shows a conventional leadless pacemaker 20 as explained above.

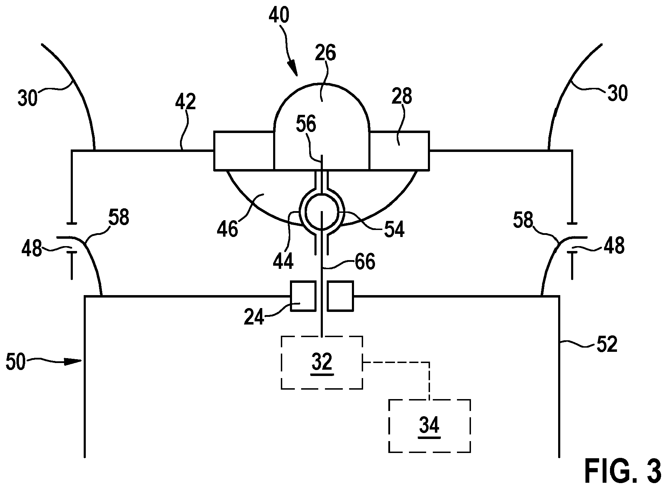

Figure 2 shows a port 40 for a leadless pacemaker 50 (see figure 3). The port 40 comprises a first connector 44, which is arranged at a first side of the port 40, and at least one anchoring member 30, which is mechanically coupled to the first connector 44, for anchoring the port 40 to tissue of a heart of a patient. The first connector 44 may interact with a second connector 54 (see figure 3) of the leadless pacemaker 50 by a press fit, interference fit, positive fit, and/or bayonet mount. In particular, the leadless pacemaker 50 may be detachably attached to the port 40 via the first and second connectors 44, 54. The first side of the port 40 faces away from the tissue of the heart, if the port 40 is implanted in the body of the patient, and towards the leadless pacemaker 50, if the leadless pacemaker 50 is attached to the port 40. A second side of the port 40 faces away from the first side and the leadless pacemaker 50, if the leadless pacemaker 50 is attached to the port 40, and towards the tissue of the heart of the patient, if the port 40 is implanted in the body of the patient. The anchoring member 30 may comprise one or more tines, wires, sheets or tubes and may be anchored within the tissue of the heart.

The port 40 may further comprise a stimulation electrode 26 at the second side of the port 40. The stimulation electrode 26 may be connected to the first connector 44 by a plastic body 46. The plastic body 46 may be formed by a synthetic resin, e.g. by epoxide. The port 40 may further comprise a housing 42. The anchoring member 30 may be arranged at the housing 42 of the port 40 such that the anchoring member 30 is coupled to the first connector 44 via the housing 42. The housing 42 of the port 40 may comprise first fixation means 48 for attaching the leadless pacemaker 50 (see figure 3) to the housing 42 of the port 40. The first fixation means 48 may comprise one or more recesses within the housing 42 of the port 40. An insulation 28, which isolates the stimulation electrode 26 against the housing 42 of the port 40, may be provided between the housing 42 and the stimulation electrode 26. The insulation 28 may be fixed to the stimulation electrode 26 and/or the first connector 44 by the plastic body 46.

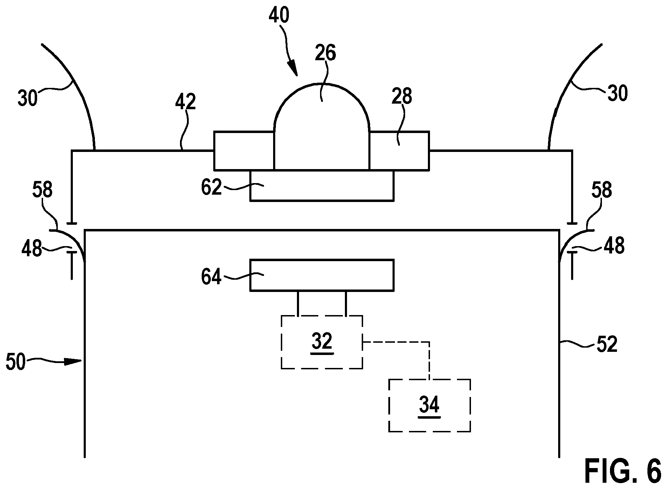

If the port 40 comprises the stimulation electrode 26, the port 40 may comprise a receiver unit for receiving a stimulation signal from the leadless pacemaker 50 and for forwarding the stimulation signal to the stimulation electrode 26. In the simplest case, the receiver unit may comprise a conductive body only, e.g. a first wire 56, for conducting the received stimulation signal to the stimulation electrode 26. The first wire 56 may be arranged at least in part within the first connector 44 such that the electrical connection between the stimulation electrode 26 and the leadless pacemaker 50 is established, if the leadless pacemaker 50 is attached to the port 40 via the first connector 44. Alternatively, the receiver unit may comprise a wireless receiver, as explained below with respect to figure 6.

Fig. 3 shows a pacemaker system according to an embodiment of the present invention. The pacemaker system comprises a port 40 for a leadless pacemaker 50, e.g. the above port 40 for the leadless pacemaker 50, and a leadless pacemaker 50. The port 40 may widely correspond to the above port 40. Therefore, only those features of the port 40 of figure 3 are explained in the following, in which the port 40 of figure 3 differs from the port of figure 2.

The leadless pacemaker 50 comprises a housing 52, a stimulation circuit 32 and an energy source 34 within the housing 52, the second connector 54, and second fixation means 58 at the outside of the housing 52. The stimulation circuit 32 is configured to generate a stimulation signal for stimulating cardiac activity of the heart. The stimulation circuit 32 is electrically coupled to the energy source 34. The energy source 34 may supply energy, e.g. a current or a voltage, to the stimulation circuit 32 for generating the stimulation signal. The energy source 34 may comprise a battery.

A feedthrough 24 for guiding a second wire 66 through the housing 52 of the leadless pacemaker 50 may be formed at a first side of the leadless pacemaker 50. The feedthrough may comprise a gasket for sealing the housing 52 of the leadless pacemaker 50 against the environment. The second wire 66 may form or may belong to a transmitter unit for transmitting the stimulation signal from the stimulation circuit 32 to the receiver unit of the port 40. The second wire 66 may be arranged at least in part within the second connector 54 such that the transmitter unit of the leadless pacemaker 50 may be electrically coupled to the receiver unit of the port 40, if the first connector 44 is attached to the second connector 54. The first side of the leadless pacemaker 50 faces the port 40. A second side of the leadless pacemaker 50 faces away from the port 40.

The second connector 54 and as such the leadless pacemaker 50 is detachably attached to the port 40, in particular to the first connector 44. The second fixation means 58 may interact with the first fixation means 48 in order to fix the leadless pacemaker 50 to the housing 42 of the port 40. In particular, the second fixation means 58 may extend through and/or may be hooked into the first fixation means 48, at least in part.

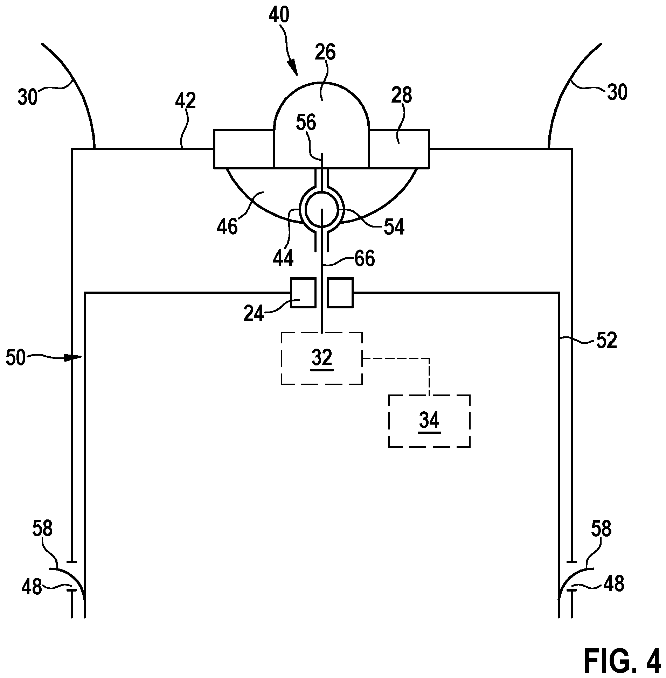

Figs. 4 -10 show various pacemaker systems according to embodiments of the present invention. Therein, the pacemaker system comprises a port 40 for a leadless pacemaker 50 and a leadless pacemaker 50, e.g. the port 40 for the leadless pacemaker 50 and the leadless pacemaker 50. For each embodiment, the port 40 and the leadless pacemaker 50 may widely correspond to the above port 40 and, respectively, leadless pacemaker 50 or to the port 40 and/or pacemaker 50 described or shown in another embodiment. Therefore, only those features of the port 40 and of the leadless pacemaker 50 are explained in the following, in which the port 40 and the leadless pacemaker 50 of the respective figure differ from the port 40 and leadless pacemaker 50 of embodiments as shown in other figures and/or as described in preceding passages.

The housing 42 of the port 40 may comprise elongated sides or side walls such that the whole or at least nearly the whole leadless pacemaker 50 may be arranged within the housing 42 of the port 40. In this case, the first fixation means 48 may be arranged adjacent an end of the elongated sides facing away from the first connector 44 and the second fixation means 58 correspondingly may be arranged adjacent the second side of the leadless pacemaker 50. This arrangement may contribute to a proper fixation of the leadless pacemaker 50 to the port 40, in particular to the housing 42 of the port 40. Further, with this construction, a tissue fibrosis and a pacemaker encapsulation (not shown in the figures) are less problematic for an exchange of the leadless pacemaker 50, as only the very back of the corresponding parts, e.g. the second side of the leadless pacemaker 50 needs to be accessible.

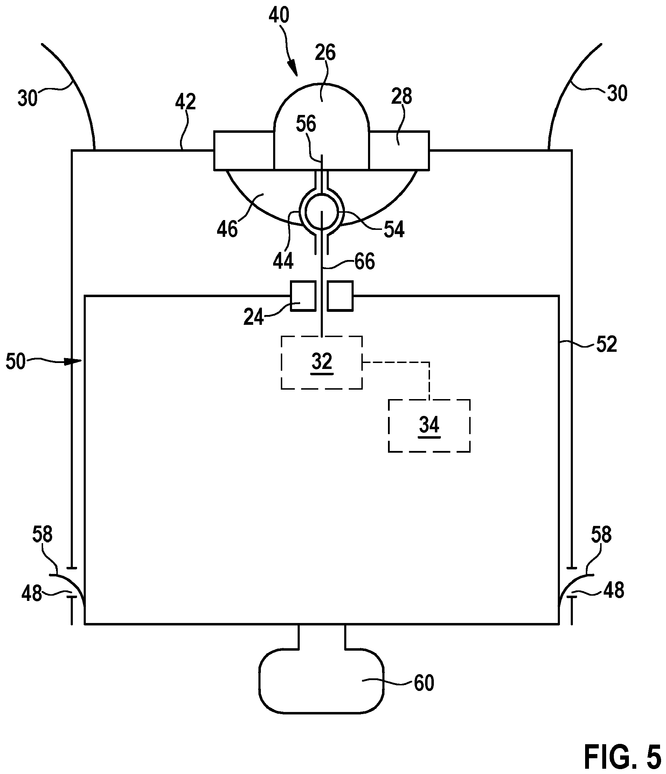

The leadless pacemaker 50 shown in figure 5 comprises retrieval means 60 at the second side of the leadless pacemaker 50. The retrieval means 60 may comprise one or more of the group of a sling, knob, loop, eyelet. The retrieval means 60 may be engaged to a detaching tool (explained below) for replacing the leadless pacemaker 50.

As shown in figure 6, the transmitter unit of the leadless pacemaker 50 comprises a wireless sender 64 for sending the wireless stimulation signal to the wireless receiver 62 of the port 40. The wireless stimulation signal may be generated by the stimulation circuit 32. The wireless sender 64 may be electrically coupled to the stimulation circuit 32 for receiving the wireless stimulation signal from the stimulation circuit 32. The receiver unit of the port 40 comprises a wireless receiver 62 for receiving the wireless stimulation signal. The wireless receiver 62 is electrically coupled to the stimulation electrode 26 for wirelessly forwarding the stimulation signal to the stimulation electrode 26.

The wireless stimulation signal may be an ultrasonic signal or an electromagnetic signal. Correspondingly, the wireless sender 64 may be an ultrasonic sender and the wireless receiver 62 may be an ultrasonic receiver, or, respectively, the wireless sender 64 may be a radio sender and the wireless receiver 62 may be a radio receiver. In case of the ultrasonic sender and receiver, the ultrasonic sender and/or the ultrasonic receiver may be micro-electro-mechanical systems (MEMS). Particularly, such ultrasonic components may be provided as piezoelectric micromachined ultrasonic transducers (PMUT).

The wireless receiver 62 and the wireless sender 64 enable to omit the feedthrough 24 through the housing 52 of the leadless pacemaker 50.

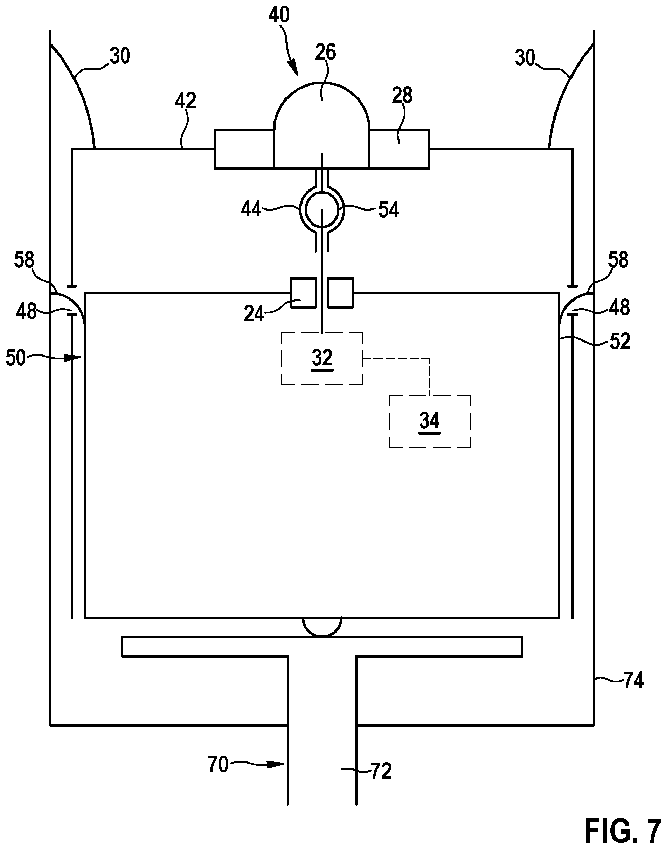

As shown in figure 7, a device for implanting the pacemaker system comprising the leadless pacemaker 50 and the port 40 within the body of the patient may comprise a catheter 70, coupling means 72 and a delivery tool 74. The delivery tool 74 is coupled to the catheter 70 by the coupling means 72. For implanting the pacemaker system, the leadless pacemaker 50 may be fixed to the port 40 by the first and second connector 44, 54 and the pacemaker system may be engaged with the delivery tool 74, which is coupled to the catheter 70. For example, the leadless pacemaker 50 may be arranged within a cavity of the delivery tool 74 and/or may be surrounded at least in part by the delivery tool 74. The catheter 70 may be introduced into the body of the patient and may be steered from outside of the body of the patient by a doctor. If the pacemaker system is implanted within the body, the delivery tool 74 may be removed from the pacemaker system and the body by the catheter 70.

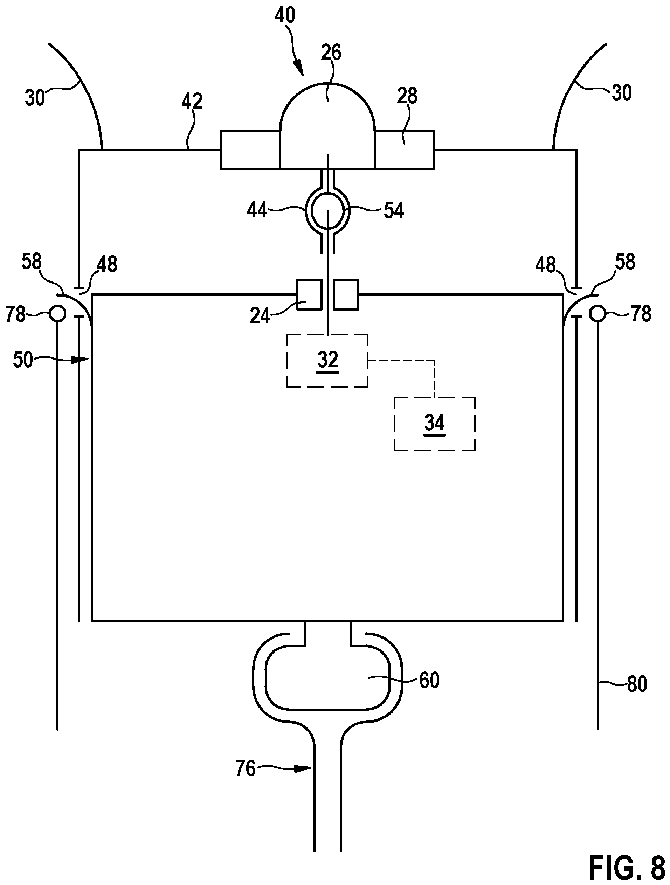

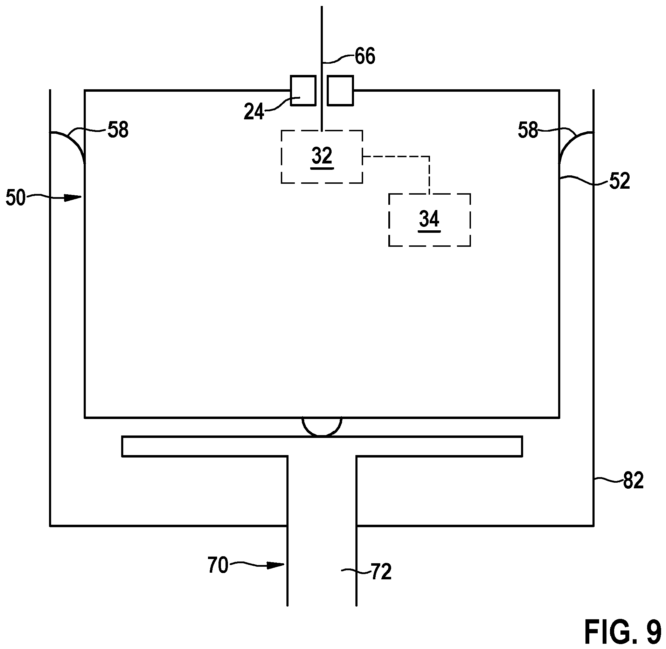

The coupling means 72 may be arranged for coupling different tools to the catheter 70, e.g. the delivery tool 74 for implanting the pacemaker system, a detaching tool 80 (see figure 8) for explanting the leadless pacemaker 50 only or a re-implanting tool 82 (see figure 9) for re-implanting the leadless pacemaker 50 only, as explained below.

As shown in figure 8, a device for removing the leadless pacemaker 50 from the body and the port 40 may comprise the above catheter 70 and the above coupling means 72 (not shown in figure 8). The device may further comprise the detaching tool 80, which may be coupled to the coupling means 72. The detaching tool 80 may comprise releasing means 78 for releasing the fixation means 48, 58 from each other and engaging means 76 for engaging the retrieval means 60 of the leadless pacemaker 50.

For detaching the leadless pacemaker 50 from the port 40, the detaching tool 80 may be introduced into the body such that the leadless pacemaker 50 is engaged with the detaching tool. In particular, the detaching tool 80 may be introduced into the body such that the leadless pacemaker 50 may be arranged at least in part within a cavity of the detaching tool 80 and/or may be surrounded at least in part by the detaching tool 80. In this position, the releasing means 78 may interact with the fixation means 48, 58 such that the leadless pacemaker 50 may be removed from the housing 42 of the port 40. The releasing means 78 may be steered and/or controlled by the doctor via the catheter 70. For example, the releasing means 78 may comprise a sling, which may be positioned next to the second fixation means 58 and which may be tightened for pressing against the second fixation means 58 via the catheter 70. At the same time, the engaging means 76 may be engaged with the retrieval means 60 of the leadless pacemaker 50. Then, the leadless pacemaker 50 may be removed from the port 40 via the engaging means 76. The engaging means 76 may be steered and/or controlled by the doctor via the catheter 70.

As shown in figure 9, a device for re-implanting the leadless pacemaker 50 only may comprise the catheter 70, the coupling means 72 and a re-implanting tool 82, which may be coupled to the coupling means 72. For re-implanting the leadless pacemaker 50 into the body and attaching it to the port 40, the re-implanting tool 82 may be loaded with the leadless pacemaker 50. In particular, the leadless pacemaker 50 may be arranged at least in part within a cavity of the re-implanting tool 82 and/or may be surrounded at least in part by the re-implanting tool 82. Then, the re-implanting tool 82 with the leadless pacemaker 50 may be introduced into the body such that the leadless pacemaker 50 is engaged with the port 40, i.e. such that the first connector 44 is connected with the second connector 54. In this position, the re-implanting tool 82 may be disengaged from the leadless pacemaker 50. The re-implanting tool 82 may be steered and/or controlled by the doctor via the catheter 70.

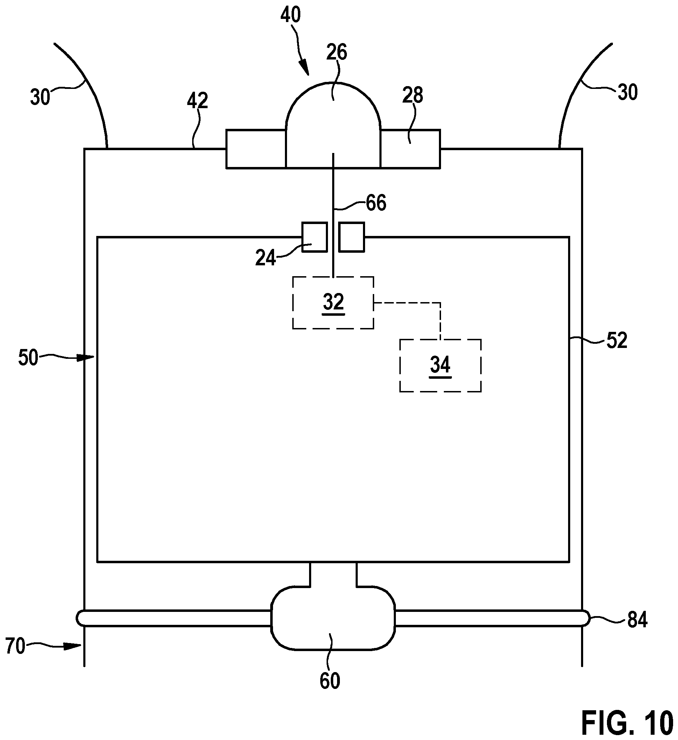

As shown in figure 10, the pacemaker system may comprise alternative fixation means 84. The alternative fixation means 84 may comprise a butterfly lock. A first part of the butterfly lock may be arranged at the housing 52 of the leadless pacemaker 50 and a second part of the butterfly lock, which may interact with the first part of the butterfly lock, may be arranged at the housing 42 of the port 40.

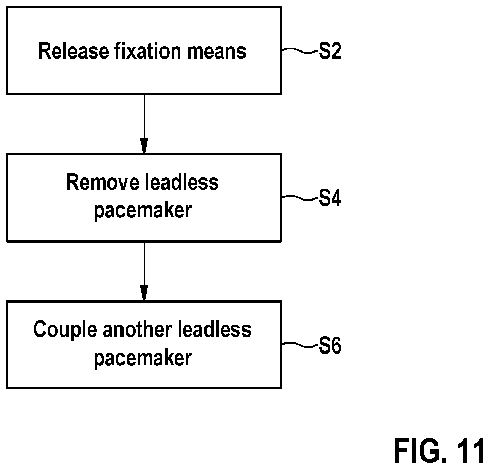

Fig. 11 shows a flow diagram of a method for exchanging a leadless pacemaker according to an embodiment of the present invention, e.g. one of the above leadless pacemakers 50. The method may be carried out, if the leadless pacemaker 50 is implanted in the heart of the patient and does not work properly anymore, e.g. because the stimulation circuit 32 has a malfunction and/or because the energy source 34 is exhausted. Then, the leadless pacemaker 50 may be removed and substituted by another leadless pacemaker as explained in the following.

In advance, the body of the patient may be opened, a detaching tool, e.g. the above detaching tool 80, may be introduced into the body and may be guided to the leadless pacemaker 50, which is coupled to the port 40, wherein the port 40 is already implanted within the tissue of the heart.

In step S2, fixation means for fixing the leadless pacemaker 50 to the port 40, e.g. the above fixation means 48, 58, may be released, e.g. by the releasing means 78 of the detaching tool 80.

In step S4, the leadless pacemaker 50 may be removed from the port 40 and from the body of the patient.

In step S6, another leadless pacemaker 50, which works properly and/or which may comprise a new and/or full energy source may be re-implanted and may be attached to the port 40, which stays at its place during the whole procedure.

The invention is not restricted to the above embodiments. For example, the above embodiments may be combined. For example, each of the above embodiments may comprise or may not comprise the first and second wire 56, 66, or the wireless sender and receiver 62, 64 for transmitting the stimulation signal. Further, each of the above embodiments may comprise or may not comprise the port 40 with the elongated sides as described with respect to figure 4, and/or the retrieval means 60 as described with respect to figure 5. Furthermore, in each of the above embodiments the stimulation electrode 26 may be arranged at the port 40 or at the leadless pacemaker 50.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of Reference Numerals

- 20: conventional leadless pacemaker
- 22: conventional housing
- 24: feedthrough
- 26: stimulation electrode
- 28: insulation
- 30: anchoring member
- 32: stimulation circuit
- 34: energy source
- 40: port
- 42: housing of port
- 44: first connector
- 46: plastic body
- 48: first fixation mean
- 50: leadless pacemaker
- 52: housing of leadless pacemaker
- 54: second connector
- 56: first wire
- 58: second fixation mean
- 60: retrieval means
- 62: wireless receiver
- 64: wireless sender
- 66: second wire
- 70: catheter
- 72: coupling means
- 74: delivery tool
- 76: engaging means
- 78: releasing means
- 80: detaching tool
- 82: re-implanting tool
- 84: alternative fixation means
- S2-S6: steps two to four

## Claims

1. Port (40) for a leadless pacemaker (50) for stimulating cardiac activity of a heart of a patient, the port (40) comprising:
a first connector (44), which is arranged at a first side of the port (40) and which is configured for mechanically and detachable attaching the leadless pacemaker (50) to the port (40); and
at least one anchoring member (30), which is mechanically coupled to the first connector (44), for anchoring the port (40) to tissue of the heart.

2. Port (40) of claim 1, comprising:
a stimulation electrode (26) at a second side of the port (40), the second side facing away from the first side; and
a receiver unit, which is electrically coupled to the stimulation electrode (26) and
which is configured for receiving a stimulation signal from the leadless pacemaker (50) and to forward the stimulation signal to the stimulation electrode (26),
wherein the stimulation electrode (26) is configured to stimulate the cardiac activity depending on the stimulation signal.

3. Port (40) of claim 2, wherein
the stimulation signal from the leadless pacemaker (50) is a wireless signal and
the receiver unit is a wireless receiver (62) configured for receiving the wireless stimulation signal.

4. Port (40) of one of claims 1 to 3, further comprising:
a housing (42), which surrounds a cavity for accommodating at least a part of the leadless pacemaker (50) and which has a recess extending through the housing (42) and in which at least a part of the first connector (44) and/or the stimulation electrode (26) are arranged, and/or
an insulation (28), which is coupled to the housing (42) and which is configured to electrically insulating the stimulation electrode (26) against the housing (42).

5. Port (40) of claim 4, wherein
the anchoring member (30) is arranged at the housing (42).

6. Leadless pacemaker (50) for stimulating cardiac activity of a heart of a patient, the leadless pacemaker (50) comprising:
a second connector (54), which is arranged at a first side of the leadless pacemaker (50) and which is configured for mechanically and detachable attaching the leadless pacemaker (50) to a port (40) for the leadless pacemaker (50), the port (40) being attached to tissue of the heart;
a stimulation circuit (32), which is configured for generating a stimulation signal, and
an energy source (34), which is electrically coupled to the stimulation circuit (32) and which is configured to supply energy to the stimulation circuit (32).

7. Leadless pacemaker (50) of claim 6, comprising:
a transmitter unit, which is electrically coupled to the stimulation circuit (32) and
which is configured for sending the generated stimulation signal to a receiver unit of the port (40).

8. Leadless pacemaker (50) of claim 7, wherein
the stimulation signal is a wireless signal and
the transmitter unit is a wireless sender (64) configured for wirelessly sending the stimulation signal to the wireless receiver (62) of the port (40).

9. Leadless pacemaker (50) of claim 6, comprising:
a stimulation electrode (26) at the first side of the leadless pacemaker (50), wherein
the stimulation electrode (26) is electrically coupled to the stimulation circuit (32) for receiving the stimulation signal and wherein the stimulation electrode (26) is arranged and configured such that the cardiac activity of the heart of the patient can be stimulated by the stimulation electrode (26), if the port (40) is attached to the tissue of the heart and if the leadless pacemaker (50) is mechanically coupled to the port (40).

10. Leadless pacemaker (50) of one of claims 6 to 9, comprising:
a housing (52), and
fixation means (58), which are arranged at the housing (52) of the leadless pacemaker (50) and which are configured for detachable attaching the housing (52) of the leadless pacemaker (50) to the port (40).

11. Leadless pacemaker (50) of one of claims 6 to 10, comprising:
retrieval means (60) at a second side of the leadless pacemaker (50), the second side of the leadless pacemaker (50) facing away from the first side of the leadless pacemaker (50).

12. Pacemaker system, comprising:
the port (40) in accordance with one of claims 1 to 5 and
the leadless pacemaker (50) in accordance with one of claims 6 to 11.

13. Pacemaker system of claim 12, wherein
the leadless pacemaker (50) is mechanically and detachable attached to the port (40).

14. Detaching tool (80) for detaching a leadless pacemaker (50) from a port (40), which is attached to tissue of a heart of a patient, particularly for detaching the leadless pacemaker (50) in accordance with one of claims 6 to 11 from the port (40) in accordance with one of claims 1 to 5, the detaching tool (80) comprising:
releasing means (78), which are configured for interacting with fixation means (58) at a housing (52) of the leadless pacemaker (50) such that the fixation means (58) may be released by the releasing means (78);
engaging means (78), which are coupled to the releasing means (78) and which are configured to engage with retrieval means (60) of the leadless pacemaker (50) such that the leadless pacemaker (50) may be removed from the port (40) by the detaching tool (80); and
coupling means (72), which are coupled to the releasing means (78) and/or the engaging means (78), and which are configured for coupling the detaching tool (80) to a catheter (70).

15. Method for exchanging a leadless pacemaker (50), which is attached to a port (40), the port (40) being attached to tissue of a heart of a patient, the method comprising:
engaging the leadless pacemaker (50) and releasing fixation means (58), with which the leadless pacemaker (50) is mechanically coupled to the port (40);
removing the leadless pacemaker (50) from the port; and
coupling another leadless pacemaker (50) to the port (40).
